# EUROPEAN PATENT APPLICATION

(11) **EP 1 020 531 A2**
(43) Date of publication of application: **19.07.2000**
(21) Application number: 99310633.5
(22) Date of filing: 30.12.1999
(51) Int. Cl.: C12Q 1/18

(54) **Use of spore adhesion assays in fungicide screening**

(30) Priority: 13.01.1999 US 115851 P
(71) Applicant: ROHM AND HAAS COMPANY, Philadelphia, Pennsylvania 19106-2399 (US)
(72) Inventor: Young, David Hamilton, Ambler, Pennsylvania 19002 (US); Slawecki, Richard Andrew, Warminster, Pennsylvania 18974 (US)
(74) Representative: Buckley, Guy Julian

(57) **Abstract**

This invention provides a method for screening compounds for fungitoxicity based on measurement of the ability to inhibit the germination-associated adhesion of fungal spores to a substrate. The method is useful for detecting compounds which inhibit the germination of fungal spores.

## Description

Fungi are the causal agents of many important diseases of plants and humans, and also cause biodeterioration of materials such as wood, paint and plastic. There is a continuing need to discover new antifungal compounds which are more efficaceous than existing products, and have more favorable environmental and toxicological properties.

To increase the chance of success in discovering new antifungal compounds with favorable properties it is desirable to screen large numbers of compounds as efficiently as possible. Frequently, the amount of test compound available is small, necessitating the use of some form of "in vitro" screen in which fungitoxicity is evaluated in a microtiter plate or other small vessel, rather than testing each compound for ability to control fungal disease on plants.

Many different techniques are available for measuring fungitoxicity "in vitro". Common methods involve measurement of growth inhibition zones produced by the test compound on agar plates seeded with fungus, measurement of fungal colony diameters in nutrient medium amended with the test compound, and visual or spectrophotometric evaluation of myelial growth in a microtiter plate or other vessel containing nutrient medium amended with the test compound.

Germination tests in which compounds are evaluated for their ability to inhibit spore germination have also been used extensively for testing fungitoxicity in vitro. One advantage of this method is that spore germination occurs very rapidly for many fungi, allowing a fungitoxic effect to be measured within a few hours rather than one or more days as is typical for other methods. A further advantage is that spore germination assays can be conducted on "obligate" fungal pathogens such as rust and powdery mildew fungi which cannot be cultured on media in the laboratory. Spores of such obligate pathogens may be harvested from infected plants and induced to germinate under appropriate conditions in vitro {see for example, M.A. de Waard, *Meded. Fac. Landbouwwetensch.* **36***,* 113-119 (1971), and J. Gorska-Poczopko, *Bulletin de l'Academie Polonaise des Sciences,* **20**, 681-684 (1972)}. Germination tests are also very useful for evaluating the mechanism of action of antifungal compounds. For many compounds spore germination is the stage in fungal growth which is most sensitive to inhibition, while other fungitoxic compounds may show little or no inhibition of germination yet potently inhibit subsequent mycelial growth. Consequently, use of a germination assay along with other methods of measuring fungitoxicity such as measuring inhibition of mycelial growth or control of fungal colonization of plant, animal or material can provide valuable insight into the fungitoxic mechanism of a compound.

The major drawback of spore germination tests is the need for microscopic examination of the spores to evaluate germination, a tedious and labor intensive procedure which does not lend itself to screening large numbers of compounds. The present invention overcomes this problem by obviating the need for microscopic inspection of the spores.

The basis for the current invention is the discovery that:
1. Firm adhesion of germinating fungal spores (germination-associated adhesion) to a substrate is a general characteristic of fungi, which can be differentiated from fungal adhesion at other developmental stages.
2. Germination-associated adhesion can be inhibited by antifungal compounds.
3. The ability to inhibit germination-associated adhesion correlates well with the ability to inhibit spore germination, and therefore is a useful measurement of the ability to inhibit germination which can be employed in fungicide screening.

Consequently, measurements of germination-associated spore adhesion can provide an alternative to microscopic examination in screening compounds for their ability to inhibit germination.

The ability of fungi at various developmental stages to adhere to leaves and other substrates is well documented. Developmental stages at which adhesion has been reported to occur include spore, germinated spore, appressorial and mycelial stages {E.B.G. Jones, *Mycological Research,* 98, 961-981 (1994)}. In some situations adhesion is believed to be a passive process while in other cases adhesion involves the active excretion of adhesive materials {Braun, E.J. and Howard, R.J., *Protoplasma,* **181**, 202-212 (1994)}. Adhesion of fungal spores to surfaces prior to germination has been reported for various fungi, such as *Botrytis cinerea* {R.P. Doss et al., *Applied and Environmental Microbiology,* **59,** 1786-1791 (1993)}, *Magnaporthe grisea* {Hamer, J.E. et al., Science, 239, 288-290 (1988)}, *Colletotrichum lindemuthianum* {Young, D.H. and Kauss, H., Applied and Environmental Microbiology, 47, 616-619 (1984)}, and *Colletotrichum graminicola* {Mercure, E.W. et al., *Physiological and Molecular Plant Pathology,* 46, 121-135 (1995)}. Spore adhesion during or shortly after germination has been reported for fungi such as *Botrytis cinerea* {R.P. Doss et al., *Applied and Environmental Microbiology,* **61**, 260-265 (1995)}, *Puccinia sorghi* {Chaubal, R. et al., *Canadian Journal of Botany,* **69,** 2044-2054 (1991)}, and *Uromyces appendiculatus* {L. Epstein et al., *Physiological and Molecular Plant Pathology,* **30***,* 373-388 (1987)}. Because adhesion for various fungi has been reported to occur both prior to and following germination it is surprising that adhesion would provide the basis for a screening method for fungicides which inhibit germination.

Inoue, et al. *{Pesticide Science,* **19**, 145-152 (1987)} reported that chlobenthiazone and tricyclazole inhibited adhesion of *Pyricularia oryzae* appressoria to barley leaves. However, appressoria are specialized structures formed by certain fungi at a later stage than germination to aid penetration. Furthermore, chlobenthiazone and tricyclazole act by inhibiting melanization in fungi, have little effect on fungal growth or germination, and would not be detectable using the method of the present invention. Vuddhakel et al., *{Journal of Antimicrobial Chemistry,* **21**, 755-763 (1988)} reported that adhesion of the yeast form of *Candida albicans,* a human pathogenic fungus, to dacron fibers is inhibited by various antimycotic drugs. This report does not relate to the current invention since the yeast form of *Candida albicans* grows by a budding mechanism rather than by germ tube development as in the case of the fungi employed in the method of the present invention. Significantly, azole antifungals and amorolfine were found to inhibit *Candida* adherence whereas these compounds, as inhibitors of ergosterol biosynthesis, are known to have little effect on germination of fungal spores and to act instead on subsequent mycelial growth.

The present invention provides a method for the determination of the antifungal activity of potential fungicidal compounds against fungi which produce spores or conidia capable of germination, said method comprising the steps of
(i) applying a suspension of fungal spores in water or an aqueous nutrient medium to the surface of a substrate in the presence (treated) and absence (control) of the potential fungicidal compound,
(ii) incubating at a temperature which favors spore germination sufficiently long for germination to occur,
(iii) removing the unbound spores,
(iv) measuring the number of spores or amount of fungal material bound to the surface of the substrate in the presence and absence of the potential fungicidal compound, and
(v) identifying a fungitoxic effect of the potential fungicide as indicated by a reduction in the number of spores or amount of fungal material bound to the surface in the treated sample as compared with the control.

In an alternative embodiment, the present invention provides a method for the determination of the antifungal activity of potential fungicidal compounds against fungi which produce spores or conidia capable of germination, said method comprising the steps of
(i) applying a suspension of fungal spores in water or an aqueous nutrient medium to the surface of a substrate in the presence (treated) and absence (control) of the potential fungicidal compound,
(ii) incubating at a temperature which favors spore germination sufficiently long for germination to occur,
(iii) determining the number of spores or amount of fungal material bound to the surface of the substrate in the presence and absence of the potential fungicide compound by measuring the number of spores or amount of fungal material which fails to bind to the substrate in the treated and control samples and subtracting this from the total number of spores or total amount of fungal material, and
(iv) identifying a fungitoxic effect of the potential fungicide as indicated by a reduction in the number of spores or amount of fungal material bound to the surface in the treated sample as compared with the control.

The number of spores or amount of fungal material bound to the substrate can be quantified by any appropriate technique. Similar techniques can be used to estimate adhesion based on measurement of the number of spores or amount of fungal material in the unbound spore population. Examples of techniques which can be used to quantify bound or unbound spores or fungal material include, but are not necessarily limited to:
a) microscopic analysis by visual inspection or with the aid of image analysis equipment,
b) use of cell counting equipment, e.g. a Coulter Counter®,
c) spectrophotometric, fluorimetric or luminescence techniques to quantify spores based on intrinsic colored, fluorescent or luminescent cellular components,
d) staining of cellular components of the spores either before or after adhesion with the use of spectrophotometric, fluorimetric or luminescence measurements, as appropriate for the particular stain, to quantify adhesion,
e) chemical analysis, and
f) labelling of cellular components of the spores either before or after adhesion with a radioactive material and quantitation of spore adhesion by measurement of the bound radioactivity.

It is also understood that the fungus itself may be manipulated to facilitate the detection of adhered spores in the adhesion assay. For example, fungi may by genetically modified to express an easily detectable signal such as the green fluorescent protein, pigments or fluorochromes.

Examples of substrates which can be employed in spore adhesion assays include, but are not necessarily limited to, polystyrene, polyethylene, polypropylene, polymethylpentene (Permanox®), polymethylmethacrylate (Plexiglas®) and glass. Assays can also be conducted using a natural or synthetic substrate capable of colonization by the organism, such as plant or animal cells and tissue, leather, wood, textiles, metal or plastic.

The method of the present invention can be applied to screening for antifungal activity against Ascomycete, Basidiomycete and Deuteromycete fungi which produce spores or conidia capable of germination. Examples include, but are not necessarily limited to, *Botrytis* spp., *Magnaporthe* spp., *Colletotrichum* spp., *Aspergillus* spp., *Puccinia* spp., *Uromyces spp., Erysiphe* spp., *Fusarium* spp., *Alternaria* spp., *Penicillium* spp., *Helminthosporium* spp., *Venturia* spp., *Cercospora* spp., *Septoria* spp., *Mycosphaerella* spp., *Uncinula* spp., *Podosphaera* spp., *Monilinia* spp., *Sphaerotheca* spp., *Pyrenophora* spp., *Pseudocercosporella* spp., *Rhyncosporium* spp., *Sclerotinia* spp., and *Phoma* spp.

The following examples are provided for further guidance to the practitioner of this invention.

### Example 1. Temporal correlation between spore adhesion and germination for Botrytis cinerea

*Botrytis cinerea* was grown at room temperature under fluorescent lights on potato dextrose agar (PDA), and subcultured every 14 days. To prepare spore suspensions, 20 ml sterile milliQ™ water was added to plates (10-14 days old) and the surface scraped gently with a sterile loop. The fluid was filtered through glass wool to remove mycelial fragments, then the spore density was adjusted to the desired concentration.

Assays were performed in polymethylpentene chamber slides (2-well Lab-Tek Permanox® chamber slides, Nunc Inc.). Chamber slide wells received either 5 µl of dimethyl sulfoxide (DMSO) only (the controls) or test compound dissolved in DMSO, followed by 500 µl of Sabouraud dextrose broth (SDB, obtained from Difco Laboratories). Aqueous spore suspension (500 µl) at 200,000 spores/ml was added, mixed gently and the vials or slides incubated at 25°C for the desired period. To evaluate spore adhesion, the number of nonadhered spores was determined by transferring the fluid to a counting vial, washing the chamber slide well once by adding 1 ml of Isoton® II counting fluid to the well, shaking gently, and then adding the wash to the counting vial. Counting fluid (14 ml) was added to the vial, and the spores counted using a Coulter Counter® (Coulter Corporation Scientific Instruments). The number of adhered spores was determined by subtracting the number of nonadhered spores from the total number of spores and was expressed as a percentage (% Adhesion). To evaluate germination, the total spore population in the chamber slides was fixed in 5% glutaraldehyde by adding 0.077 ml of 70% glutaraldehyde to chamber slide wells. The number of germinated spores was then determined by microscopic examination of a minimum of 100 spores for evidence of germ tube emergence. The number of germinated spores was expressed as a percentage of the total (% Germination). Data in the table below show a time-dependent adhesion of spores to the slide which closely parallels the time-course for spore germination.

| Time (hours) | %Adhesion | %Germination |
|---|---|---|
| 0 | 0 | 0 |
| 1 | 14.1 | 5.3 |
| 2 | 30.4 | 27.0 |
| 3 | 78.6 | 83.2 |
| 4 | 91.0 | 89.3 |
| 6 | 93.8 | 95.8 |
| 8 | 96.5 | 98.8 |

### Example 2. Effect of fungicides on germination-associated adhesion of Botrytis cinerea spores

Various fungicides were evaluated for their abilities to inhibit germination-associated spore adhesion to polystyrene counting vials, polymethylpentene (Permanox®) chamber slides and 96-well flat-bottomed polystyrene microtiter plates, and for their effects on spore germination and mycelial growth (table below). Adhesion assays in chamber slides and germination assays were performed as in Example 1 with incubation at 25°C for 5.5 h. Adhesion assays in counting vials were conducted in 20 ml capacity polystyrene counting vials (VWR cat. no. 14310-640). Counting vials received 5 µl of DMSO (controls) or test compound dissolved in DMSO, followed by 500 µl of SDB. Aqueous spore suspension (500 µl) at 200,000 spores/ml was added, mixed gently and the vials or slides incubated at 25°C for 5.5 h. To determine the numbers of unbound spores 15 ml of counting fluid was added, vials were capped and inverted gently 5 times, then counted using a Coulter Counter®.

For adhesion assays in microtiter plates, stock solutions of test compounds were prepared in DMSO then diluted in SDB such that the DMSO concentration after dilution was ≤2%. Two-fold serial dilutions of these test compound solutions were made in SDB (100 µl) in 96-well flat-bottomed microtiter plates. Spore suspension (100 µl at 2X10⁵ spores/ml) was added to the wells, and the plates were incubated at 25°C for 5.5 hours. The medium containing unbound spores was removed by inverting and flicking the microtiter plate to remove as much liquid as possible. Quantitation of the adhered spores was achieved by measuring their cellular protein content using sulforhodamine B {(Skehan et al., *Journal of the National Cancer Institute,* **82***,* 1107-1112 (1990)}, measuring absorbance at 564 nm. Inhibition of adhesion was determined by comparing the absorbance value in a well containing fungicide with the absorbance in control wells without fungicide. EC50 values were determined from dose-response curves.

Inhibition of mycelial growth was evaluated in poison agar assays. A 4-fold dilution series of each fungicide to be tested was prepared in DMSO and 125 µl of each dilution was added to 25 ml of molten malt extract agar (20 g malt extract, 20 g glucose, 1 g peptone and 20 g agar per liter). The medium containing test compound was poured immediately into 9 cm diameter petri dishes. Two replicate plates were used for each treatment. Each plate was inoculated with a 7 mm diameter plug taken from the growing edge of a *Botrytis* culture on PDA. Colony diameters were measured after growth for 3 days in the dark at 25°C. EC50 values were determined from dose-response curves.

Results are shown in the table below. For each compound tested, EC50 values from the 3 adhesion assays were similar. Spore adhesion was sensitive to all compounds which inhibited germination (germination inhibitors, GI) at low concentrations (<2 ppm). In contrast, compounds which did not inhibit germination at low concentrations (compounds acting after germination, AG), and for which mycelial growth was much more sensitive to inhibition than germination, had little or no activity in the spore adhesion assay. EC50s for inhibition of adhesion were generally comparable to EC50s for inhibition of germination.

| | | Inhn. of Spore Adhesion (EC50, ppm) | | | | |
|---|---|---|---|---|---|---|
| Compound | *Class | Counting vials | Microtiter plates | Chamber Slides | Inhn. of Germination EC50 (ppm) | Mycelial Growth Inhn. EC50 (ppm) |
| Kresoxim-methyl | GI | 0.014 | 0.011 | 0.035 | 0.020 | 0.28 |
| Azoxystrobin | GI | 0.19 | 0.19 | 0.28 | 0.32 | 3.05 |
| Chlorothalonil | GI | 0.093 | 0.10 | 0.089 | 0.067 | 0.35 |
| Folpet | GI | 0.21 | 0.33 | 0.22 | 0.15 | 5.50 |
| Thiram | GI | 1.04 | 1.86 | 1.87 | <0.625 | 0.78 |
| Fluazinam | GI | 0.041 | 0.051 | 0.071 | 0.028 | 0.033 |
| Fenpiclonil | GI | 0.096 | 0.090 | 1.89 | <0.0312 | 0.0015 |
| Vinclozolin | GI | 0.56 | 0.62 | >5.0 | <0.5 | 0.11 |
| Carbendazim | AG | >25 | >10 | >25 | >25 | 0.045 |
| Pyrimethanil | AG | >25 | >10 | >25 | 8.6 | 0.48 |

| | | | | | | |
|---|---|---|---|---|---|---|
| *GI=inhibitor of germination, AG=compound acting after germination. | | | | | | |

### Example 3. Effect of kresoxim-methyl on germination-associated adhesion of Botrytis cinerea spores to Plexiglas® slides.

Assays were conducted in custom-made chamber slides made of Plexiglas®. Chamber slide wells received 5 µl of DMSO (controls) or kresoxim-methyl dissolved in DMSO, followed by 500 µl of SDB. Aqueous spore suspension (500 µl) at 200,000 spores/ml was added, mixed gently and the vials or slides incubated at 25°C for the desired period. The number of nonadhered spores was determined by transferring the fluid to a counting vial, washing the chamber slide well once by adding 1 ml of counting fluid to the well, shaking gently, and then adding the wash to the counting vial. Counting fluid (14 ml) was added to the vial, and the spores counted using a Coulter Counter®. The number of adhered spores was determined by subtracting the number of nonadhered spores from the total number of spores and was expressed as a percentage (% Adhesion). The results (table below) demonstrate the adhesion of germinating *Botrytis* spores to Plexiglas® and the ability of kresoxim-methyl to inhibit adhesion.

| Time (hours) | %Adhesion in control | %Adhesion with kresoxim-methyl, 10 ppm |
|---|---|---|
| 0 | 0 | |
| 1 | 2.0 | |
| 2 | 8.4 | |
| 3 | 14.4 | |
| 4 | 25.8 | |
| 5 | 49.1 | |
| 6 | 64.3 | |
| 7 | 83.6 | 17.4 |
| 8 | 89.6 | 26.2 |

### Example 4. Effect of kresoxim-methyl on germination-associated adhesion of Botrytis cinerea spores to glass vials.

Assays were conducted in 27 X 55 mm borosilicate glass vials, obtained from Kimble Glass Inc. The vials received 5 µl of DMSO (controls) or kresoxim-methyl dissolved in DMSO, followed by 500 µl of SDB. Aqueous spore suspension (500 µl) at 200,000 spores/ml was added, mixed gently and the vials incubated at 25°C for the desired period. To determine the numbers of unbound spores 15 ml of counting fluid was added, vials were capped and inverted gently 5 times, then counted using a Coulter Counter®. The number of adhered spores was determined by subtracting the number of nonadhered spores from the total number of spores and was expressed as a percentage (% Adhesion). The results (table below) demonstrate the adhesion of germinating *Botrytis* spores to glass and the ability of kresoxim-methyl to inhibit adhesion.

| Time (hours) | %Adhesion in Control | %Adhesion with kresoxim-methyl, 10 ppm |
|---|---|---|
| 0 | 0 | |
| 2 | 2.7 | |
| 3 | 4.6 | |
| 4 | 26.3 | |
| 5 | 58.8 | |
| 6 | 72.6 | 23.4 |
| 7 | 83.4 | 29.6 |

### Example 5. Effect of kresoxim-methyl on germination-associated adhesion of Botrytis cinerea spores to polyethylene vials.

Assays were conducted in 26.9 X 59.5 mm polyethylene vials (PolyQ vials, obtained from Beckman Instruments, Inc.). The vials received 5 µl of DMSO (controls) or kresoxim-methyl dissolved in DMSO, followed by 500 µl of SDB. Aqueous spore suspension (500 µl) at 200,000 spores/ml was added, mixed gently and the vials incubated at 25°C for the desired period. To determine the numbers of unbound spores 15 ml of counting fluid was added, vials were capped and inverted gently 5 times, then counted using a Coulter Counter®. The number of adhered spores was determined by subtracting the number of nonadhered spores from the total number of spores and was expressed as a percentage (% Adhesion). The results (table below) demonstrate the adhesion of germinating *Botrytis* spores to polyethylene and the ability of kresoxim-methyl to inhibit adhesion.

| Time (hours) | %Adhesion in Control | %Adhesion with kresoxim-methyl, 10 ppm |
|---|---|---|
| 0 | 0 | |
| 2 | 9.8 | |
| 3 | 20.4 | |
| 4 | 36.8 | |
| 5 | 60.9 | |
| 6 | 74.9 | 12.7 |
| 7 | 89.0 | 15.1 |

### Example 6. Effect of kresoxim-methyl on germination-associated adhesion of Botrytis cinerea spores to polypropylene vials.

Assays were conducted in 25 X 56 mm RediPak® Starline polypropylene vials, obtained from Wheaton Scientific Products. The vials received 5 µl of DMSO (controls) or kresoxim-methyl dissolved in DMSO, followed by 500 µl of SDB. Aqueous spore suspension (500 µl) at 200,000 spores/ml was added, mixed gently and the vials incubated at 25°C for the desired period. To determine the numbers of unbound spores 15 ml of counting fluid was added, vials were capped and inverted gently 5 times, then counted using a Coulter Counter®. The number of adhered spores was determined by subtracting the number of nonadhered spores from the total number of spores and was expressed as a percentage (% Adhesion). The results (table below) demonstrate the adhesion of germinating *Botrytis* spores to polypropylene and the ability of kresoxim-methyl to inhibit adhesion.

| Time (hours) | %Adhesion in Control | %Adhesion with kresoxim-methyl, 10 ppm |
|---|---|---|
| 0 | 0 | |
| 2 | 7.0 | |
| 3 | 25.0 | |
| 4 | 25.9 | |
| 5 | 59.3 | |
| 6 | 59.5 | |
| 7 | 63.2 | 18.9 |

### Example 7. Temporal correlation between spore adhesion and germination for Colletotrichum lagenarium

*Colletotrichum lagenarium* was grown at 25°C in the dark on potato dextrose agar and subcultured every 7-14 days. To prepare spore suspensions, 15 ml sterile water was added to plates (7-10 days old) and the plates agitated gently on a gyrotary shaker for 10 min to release spores. The resulting spore suspension was filtered through glass wool and adjusted to the desired concentration. Spore adhesion assays and germination assays were conducted as outlined in Example 1 for *Botrytis* except that the SDB medium contained Tween™ 20 (40 ppm), and the spore inoculum was prepared at 5X10⁵ spores/ml. Data in the table below show a time-dependent adhesion of spores to the slide which closely parallels the time-course for spore germination.

| Time (hours) | %Adhesion | %Germination |
|---|---|---|
| 0 | 0 | 0 |
| 2 | 17.1 | 0 |
| 4 | 32.8 | 11.8 |
| 6 | 85.8 | 50.5 |
| 8 | 96.4 | 97.7 |

### Example 8. Effect of fungicides on adhesion of Colletotrichum lagenarium spores

Various fungicides were evaluated for their abilities to inhibit spore adhesion in 96-well flat-bottomed polystyrene microtiter plates and Permanox® chamber slides, and for their effects on spore germination and mycelial growth. Assays were conducted as outlined in Example 2 for *Botrytis* except that the SDB medium contained Tween™ 20 (40 ppm), the spore inoculum was prepared at 5X10⁵ spores/ml, and and 8 h incubation was used for the adhesion and germination assays. Results are shown in the table below. For each compound tested, EC50 values for inhibition of adhesion to microtiter plates and chamber slides were similar. Spore adhesion was sensitive to all compounds which inhibited germination at low concentrations (<0.2 ppm). In contrast, carbendazim which did not inhibit germination, and for which mycelial growth was much more sensitive to inhibition than germination, had little or no activity in the spore adhesion assay. EC50s for inhibition of adhesion were generally comparable to EC50s for inhibition of germination.

| | | Inhn. of Adhesion (EC50, ppm) | | | |
|---|---|---|---|---|---|
| Compound | *Class | Microtiter plates | Chamber slides | Germination EC50 (ppm) | Mycelial Growth Inhn. EC50 (ppm) |
| Kresoxim-methyl | GI | 0.026 | 0.054 | 0.015 | >10 |
| Azoxystrobin | GI | 0.027 | 0.014 | 0.010 | >10 |
| Chlorothalonil | GI | 0.068 | 0.104 | 0.005 | 7.10 |
| Fluazinam | GI | <0.019 | 0.007 | 0.004 | 0.017 |
| Carbendazim | AG | 1.96 | >10 | >10 | 0.060 |

| | | | | | |
|---|---|---|---|---|---|
| *GI=inhibitor of germination, AG=compound acting after germination. | | | | | |

### Example 9. Effect of fungicides on adhesion of Aspergillus nidulans spores

Various fungicides were evaluated for their abilities to inhibit spore adhesion in 96-well flat-bottomed polystyrene microtiter plates. Adhesion assays were conducted as outlined in Example 2 for *Botrytis* except that the spore inoculum was prepared at 2X10⁶ spores/ml, and plates were incubated at 37°C for 8 h. As shown in the table below, germination inhibitors (kresoxim-methyl, fluazinam and chlorothalonil) were highly potent inhibitors of spore adhesion, whereas benomyl, which acts after germination and is a highly potent inhibitor of mycelial growth, was not.

| **Compound** | ***Class** | **Spore Adhesion Assay EC50** |
|---|---|---|
| kresoxim-methyl | GI | <0.039 |
| chlorothalonil | GI | 0.078 |
| fluazinam | GI | 0.097 |
| benomyl | AG | >20 |

| | | |
|---|---|---|
| *GI=inhibitor of germination, AG=compound acting after germination. | | |

### Example 10. Temporal correlation between spore adhesion and germination for Magnaporthe grisea

Spores were harvested from petri dish cultures of *Magnaporthe grisea* grown on potato dextrose agar by adding water to the plates and gently scraping the surface to dislodge the spores. The resulting suspension was filter through sterile glass wool to remove mycelial fragments, and adjusted to a concentration of 2 X 10⁵ spores/ml. The spore suspension (100 µl) was added to wells of 96-well flat-bottomed polystyrene microtiter plates containing 100 µl of yeast extract-glucose medium (YEG, 30 g glucose and 4 g yeast extract per liter of water), and the plates were incubated in the dark at 25°C. At selected times, spore germination was evaluated by fixing the spores in 5% glutaraldehyde and determining the number of germinated spores as a percentage of the total by microscopic evaluation. Separate plates were used to evaluate spore adhesion: unbound spores were removed by inverting and flicking the microtiter plate to remove as much liquid as possible. Quantitation of the adhered spores was achieved by measuring their cellular protein content using sulforhodamine B staining, and also by counting the numbers of spores in a defined area of each well at the end of the staining procedure. The data in the table below show a time-dependent adhesion of spores to the microtiter plate which closely parallels the time-course for spore germination.

| Time Course of *Magnaporthe grisea* Spore Adhesion and Germination in Microtiter Plates | | | |
|---|---|---|---|
| Time (hours) | Adhesion (A₅₆₄, SRB staining) | Adhesion (spores/mm²) | %Germination |
| 2 | 0.014 | 46.8 | 8.3 |
| 4 | 0.043 | 111.5 | 40.7 |
| 6 | 0.083 | 100.6 | 45.8 |
| 8 | 0.118 | 118.6 | 58.8 |

### Example 11. Effect of various fungicides on germination-associated adhesion of Magnaporthe grisea

Various fungicides were evaluated for their abilities to inhibit spore adhesion in 96-well falt-bottomed polystyrene microtiter plates. Stock solutions of test compounds were prepared in DMSO then diluted into YEG such that the DMSO concentration after dilution was ≤ 2%. Two-fold serial dilutions of these test compound solutions were made in YEG (100 µl) in 96-well flat-bottomed microtiter plates. Spore suspension (100 µl at 2X10⁵ spores/ml) was added to the wells and the plates were incubated in the dark at 25°C for 6 hours. Unbound spores were removed by inverting and flicking the microtiter plate to remove as much liquid as possible. Quantitation of the adhered spores was achieved by measuring their cellular protein content using sulforhodamine B staining. Inhibition of adhesion was determined by comparing the absorbance values in wells containing fungicide with the absorbance in control wells without fungicide. EC50 values for inhibition of adhesion were determined from dose-response curves. As shown in the table below, compounds which are known to inhibit spore germination such as the strobilurin-type compounds kresoxim-methyl and azoxystrobin, and multi-site fungicides (chlorothalonil and mancozeb) were potent inhibitors of germination-associated adhesion. Prochloraz, a fungicide which acts by inhibiting the biosynthesis of ergosterol, and thiophanate-methyl which disrupts cellular microtubules, showed little or no activity in the spore adhesion assay, consistent with the fact that they have little effect on germination but strongly inhibit mycelial growth.

| **Fungicide** | ***Class** | **EC50 (ppm)** |
|---|---|---|
| kresoxim-methyl | GI | 0.067 |
| azoxystrobin | GI | 0.064 |
| chlorothalonil | GI | 0.038 |
| mancozeb | GI | 0.115 |
| prochloraz | AG | >10.0 |
| thiophanate-methyl | AG | 7.5 |

| | | |
|---|---|---|
| *GI=inhibitor of germination, AG=compound acting after germination. | | |

### Example 12. Temporal correlation between spore adhesion and germination for Puccinia recondita

Spores of the wheat leaf rust pathogen *Puccinia recondita* were harvested from heavily infected wheat leaves by washing in an aqueous solution of 0.05% Tween™ 20. The resulting spore suspension was filtered through cheese cloth to remove contaminating leaf material, and diluted in 0.05% Tween™ 20 to give a concentration of 2 X 10⁵ spores/ml.

Spore suspension (50 µl at 2X10⁵ spores/ml) was added to wells of 96-well flat-bottomed polystyrene microtiter plates containing 50 µl of 0.05% Tween™ 20. The plates were allowed to sit at room temperature for 10 minutes, then incubated in the dark at 19°C with continous shaking on a gyrotary shaker at 100 rpm. At selected times, spore germination was evaluated by fixing the spores in 5% glutaraldehyde and determining the number of germinated spores as a percentage of the total by microscopic evaluation. Separate plates were used to evaluate spore adhesion: unbound spores were removed by inverting and flicking the microtiter plate to remove as much liquid as possible. Quantitation of the adhered spores was achieved by measuring their cellular protein content using sulforhodamine B staining, and also by counting the numbers of spores in a defined area of each well at the end of the staining procedure. Data in the table below show a time-dependent adhesion of spores to the slide, as measured either by sulforhodamine B staining or by counting the number of adhered spores, which closely parallels the time-course for spore germination.

| Time (hours) | Adhesion (SRB staining) | Adhesion (spores/mm²) | %Germination |
|---|---|---|---|
| 0 | 0.002 | 0 | 0 |
| 0.5 | 0.02 | 1.48 | 0 |
| 1 | 0.06 | 3.73 | 4.5 |
| 1.5 | 0.18 | 11.46 | 44.03 |
| 2 | 0.35 | 16.67 | 51.06 |
| 2.5 | 0.49 | 26.22 | 63.19 |
| 3 | 0.61 | 30.3 | 78.64 |
| 4 | 0.85 | 32.64 | 89.73 |
| 5 | 0.88 | 46.79 | 76.72 |
| 6 | 0.94 | 37.41 | 84.68 |

### Example 13. Effect of various fungicides on germination-associated adhesion of Puccinia recondita

Various fungicides were evaluated for their abilities to inhibit spore adhesion in microtiter plates. Stock solutions of test compounds were prepared in DMSO then diluted into 0.05% Tween™ 20 such that the DMSO concentration after dilution was ≤ 2%. Two-fold serial dilutions of these test compound solutions were made in 0.05% Tween™ 20 (50 µl) in 96-well flat-bottomed polystyrene microtiter plates. Spore suspension (50 µl at 2X10⁵ spores/ml) was added to the wells. The plates were allowed to sit at room temperature for 10 minutes, then incubated in the dark at 19°C for 4 hours with continous shaking on a gyrotary shaker at 100 rpm. Unbound spores were removed by inverting and flicking the microtiter plate to remove as much liquid as possible. Quantitation of the adhered spores was achieved by measuring their cellular protein content using sulforhodamine B staining. Inhibition of adhesion was determined by comparing the absorbance values for wells containing fungicide with the absorbance values for control wells without fungicide. EC50 values for inhibition of adhesion were determined from dose-response curves. As shown in the table below, compounds which are known to inhibit spore germination such as the carboxanilides (thifluzamide and carboxin), the strobilurin-type compound kresoxim-methyl, fluazinam and multi-site fungicides (chlorothalonil and mancozeb) all inhibited germination-associated adhesion. Fungicides which act by inhibiting the biosynthesis of ergosterol (fenbuconazole and myclobutanil) showed little or no activity in the spore adhesion assay, consistent with the fact that spore germination is much less sensitive to such compounds than mycelial growth.

| **Fungicide** | ***Class** | **EC50 (ppm)** |
|---|---|---|
| thifluzamide | GI | 0.043 |
| carboxin | GI | 0.081 |
| kresoxim-methyl | GI | 0.016 |
| fluazinam | GI | 0.365 |
| chlorothalonil | GI | 0.473 |
| mancozeb | GI | 0.56 |
| fenbuconazole | AG | 9.2 |
| myclobutanil | AG | >10 |

| | | |
|---|---|---|
| *GI=inhibitor of germination, AG=compound acting after germination | | |

## Claims

1. A method for the determination of the antifungal activity of potential fungicidal compounds against fungi which produce spores or conidia capable of germination, said method comprising the steps of
(i) applying a suspension of fungal spores in water or an aqueous nutrient medium to the surface of a substrate in the presence and absence of the potential fungicidal compound,
(ii) incubating at a temperature which favors spore germination sufficiently long for germination to occur,
(iii) removing the unbound spores,
(iv) measuring the number of spores or amount of fungal material bound to the surface of the substrate in the presence and absence of the potential fungicidal compound, and
(v) identifying a fungitoxic effect of the potential fungicide as indicated by a reduction in the number of spores or amount of fungal material bound to the surface in the treated sample as compared with the control.

2. A method for the determination of the antifungal activity of potential fungicidal compounds against fungi which produce spores or conidia capable of germination, said method comprising the steps of
(i) applying a suspension of fungal spores in water or an aqueous nutrient medium to the surface of a substrate in the presence and absence of the potential fungicidal compound,
(ii) incubating at a temperature which favors spore germination sufficiently long for germination to occur,
(iii) determining the number of spores or amount of fungal material bound to the surface of the substrate in the presence and absence of the potential fungicide compound by measuring the number of spores or amount of fungal material which fails to bind to the substrate in the treated and control samples and subtracting this from the total number of spores or total amount of fungal material, and
(iv) identifying a fungitoxic effect of the potential fungicide as indicated by a reduction in the number of spores or amount of fungal material bound to the surface in the treated sample as compared with the control.

3. The method of claim 1 or 2 wherein said fungi are selected from Ascomycetes, Basidiomycetes and Deuteromycetes.

4. The method of claim 3 wherein the species of fungi are selected from *Botrytis, Magnaporthe, Colletotrichum, Aspergillus, Puccinia, Uromyces, Erysiphe, Fusarium, Alternaria, Penicillium, Helminthosporium, Venturia, Cercospora, Septoria, Mycosphaerella, Uncinula, Podosphaera, Monilinia, Sphaerotheca, Pyrenophora, Pseudocercosporella, Rhyncosporium, Sclerotinia* and *Phoma.*

5. The method of claim 1 or 2 wherein said fungi may by genetically modified to express an easily detectable signal such as the green fluorescent protein, pigments or fluorochromes.

6. The method of claim 1 or 2 wherein the substrate is polystyrene, polyethylene, polypropylene, polymethylpentene, polymethylmethacrylate, glass, or a natural or synthetic substrate capable of colonization by fungi.

7. The method of claim 6 wherein the natural or synthetic substrate is plant or animal cells or tissue, leather, wood, a textile, metal or plastic.
